# DEMANDE DE BREVET EUROPEEN

(11) **EP 1 084 715 A1**
(43) Date de publication de la demande: **21.03.2001**
(21) Numéro de dépôt: 00203236.5
(22) Date de dépôt: 19.09.2000
(51) Int. Cl.: A61L 9/12

(54) **Dispositif programmable de diffusion de substance dans le milieu aérien ambiant**

(30) Priorité: 20.09.1999 FR 9911861
(71) Demandeur: Abricot SARL, 69730 Genay (FR)
(72) Inventeur: Janin, Jean-Paul, Société Aixpair-Abricot S.A.R.L., 69730 Genay (FR)
(74) Mandataire: Richebourg, Michel François

(57) **Abrégé**

La présente invention concerne un dispositif programmable de diffusion de substances dans le milieu aérien ambiant. L'appareil permet notamment de diffuser de manière contrôlée des substances odorantes ou autres en particulier des huiles de manière modulaire, ceci en fonction des variations de facteurs de l'environnement afin par exemple de procurer aux utilisateurs d'un espace, la sensation d'une ambiance parfumée constante ou bien encore d'adapter la dose de produit diffusée aux circonstances.

## Description

La présente invention concerne notamment le secteur de la diffusion de parfums d'ambiance et de produits de traitement des odeurs, mais elle ne se limite pas à la diffusion de telles substances.

Une diffusion intelligente de substances dans le milieu aérien ambiant peut s'avérer être agréable et utile tout en réduisant la quantité de substance utilisée. Une diffusion intelligente permet d'éviter les inconvénients de la diffusion d'odeur par exemple et de réduire la quantité de produit utilisée, ceci avec un effet maximum.

De nos jours, on connaît de mieux en mieux au travers d'études de plus en plus nombreuses, l'importance que peuvent revêtir les facteurs de l'environnement perceptibles ou même non perceptibles par les sens sur les comportements des animaux, des insectes mais aussi a fortiori humains.

Parmi les facteurs susceptibles d'influencer les comportements on retrouve au premier plan par exemple, les odeurs. Ainsi, de plus en plus, le facteur odeur est pris en compte dans les situations de la vie quotidienne et ceci pour un nombre de plus en plus grand d'utilisateurs et dans des lieux de plus en plus variés.

Des travaux récents ont mis en avant l'importance des odeurs ou des substances en suspension dans l'air et qui sont susceptibles d'influencer l'état physiologique ou le comportement chez les animaux mais aussi chez l'homme voir chez les végétaux. Cet effet sur l'état physiologique ou sur le comportement peut se traduire soit par exemple par une dynamisation ou au contraire favoriser un état de fatigue ou de stress sans que les individus s'en rendent forcément compte. Il peut donc être intéressant du point de vue économique de prendre en compte le facteur odeur pour le confort de travail.

Il a par ailleurs été postulé que certaines odeurs pouvaient avoir un effet apaisant alors que d'autres auraient plutôt un effet excitant ceci sans que l'on en soit conscient. Ainsi, on se rend de plus en plus compte de l'importance que peut revêtir une bonne gestion des odeurs ambiantes.

Dans le domaine de la diffusion des odeurs, il existe dans l'art antérieur quelques dispositifs notamment pour la diffusion d'odeurs, de parfums ou de fragrances.

En ce qui concerne la diffusion de substances odorantes, on connaît par exemple des dispositifs programmables de diffusions d'odeurs qui permettent de diffuser notamment des huiles essentielles sous forme de vapeurs sèches.

Dans ce type de dispositifs de l'art antérieur disponibles commercialement, la diffusion d'une substance est gérée par des horloges et des relais temporisés clignotant. Ces dispositifs connus dans l'ait antérieur permettent une programmation fixe de la diffusion des odeurs. Ils permettent notamment de programmer la date, l'heure et une intensité de diffusion fixe désirée. Une fois cette intensité de diffusion programmée, la diffusion se fait toujours avec la même intensité aux moments programmés pour la diffusion.

Ainsi, ces dispositifs de l'art antérieur permettent de programmer les jours où l'on souhaite une diffusion et dans ces jours les plages horaires de diffusion et dans ces plages de diffusion de régler une intensité de diffusion souhaitée, la même pour toutes les plages de diffusion.

Dans ces dispositifs de l'art antérieur, l'intensité de la diffusion est réglée en agissant sur le rapport entre le temps de diffusion et le temps d'arrêt du moteur qui entraîne la diffusion. Ainsi plus le temps de fonctionnement sera long, plus l'intensité de diffusion sera importante.

On s'aperçoit rapidement que ce mode de fonctionnement des dispositifs de l'art antérieur présente un inconvénient majeur, en effet pour toutes les heures de fonctionnement, il n'est pas possible de moduler les intensités de fonctionnement. Autrement dit, l'intensité de la diffusion est fixe et se situe au même niveau quel que soit le moment de fonctionnement. Les dispositifs de l'art antérieur permettent donc de régler l'intensité de la diffusion uniquement en fonction de paramètres fixes que sont les surfaces à traiter ainsi que les volumes des lieux où l'on souhaite faire agir la substance qui est diffusée.

Ces systèmes de diffusion des odeurs ne permettent pas de prendre en compte des facteurs variables de l'environnement tel qu'un flux de personnes à certaines heures, des pics de température, des variations du degré d'hygrométrie, des variations de concentration de certaines substances en fonction des heures de la journée etc.

Les systèmes de l'art antérieur qui ne peuvent s'adapter à de telles variations de l'environnement présentent vite plus d'inconvénients que d'avantages.

En effet, une diffusion non modulable aura pour inconvénient d'être soit trop forte à certains horaires, par exemple dans des lieux peu fréquentés à certaines heures, soit d'être trop faible lors de forte affluence par exemple lors des heures de pointe dans une station de métro.

Le résultat étant que dans les horaires de faible affluence par exemple, le trop plein d'odeurs incommode les personnes présentes sur les lieux et que dans les horaires de forte affluence, l'intensité des odeurs diffusées est trop faible et ne sera pas perceptible par les personnes présentes sur place. On pourra également imaginer le même problème pour des substances qui repoussent les insectes ou pour des substances anti-odeurs. En effet pour repousser les moustiques, on souhaitera une diffusion plus forte le soir.

L'inconvénient de ces appareils de diffusion d'odeur de l'art antérieur est donc une utilisation importante de produit que l'on diffuse couplée avec une efficacité faible ou inadaptée au regard de la quantité de produit utilisée et donc un coût de fonctionnement résultant élevé.

La présente invention se propose de remédier à ces inconvénients d'utilisation grâce à la possibilité de modulation de l'intensité de diffusion. Le but de ce dispositif est atteint grâce à la combinaison d'un variateur et d'un microprocesseur spécialement adapté et développé pour une diffusion intelligente et contrôlée de substances.

Le dispositif permet de programmer la diffusion d'un parfum ou d'autres substances au moment voulu mais avec une intensité adaptée aux conditions de l'environnement du moment, ceci en fonction des heures d'affluence connues ou d'autres facteurs qui peuvent varier au cours du temps, comme la température. Ainsi on pourra diffuser par exemple une odeur en fonction de l'affluence dans un lieu ou bien encore la diffusion plus importante d'une substance anti-insecte à certaines heures de la journée.

L'invention permet de régler l'intensité de diffusion en jouant sur les temps d'arrêt et de marche du moteur mais aussi sur la vitesse de fonctionnement du moteur, tout en adaptant ce temps de fonctionnement ou cette vitesse de fonctionnement du moteur au gré des heures de la journée.

En outre, l'invention comprend un moyen de réglage manuel de l'intensité de diffusion afin de s'adapter à des changements brutaux et rapides dans l'environnement et qui ne seraient pas conformes aux prévisions, ceci permettant à l'utilisateur de s'épargner une opération de programmation.

Le fonctionnement de l'invention va devenir plus clair au regard de la description détaillée d'un appareil de diffusion d'odeur selon un mode de réalisation préféré et non limitatif de l'invention.

### DESCRIPTION DETAILLEE DE L'INVENTION

La présente invention concerne in dispositif programmable de diffusion de substances dans l'atmosphère. Dans ce dessein , le dispositif comprend au moins un moyen pour stocker une ou plusieurs substances que l'on souhaite diffuser ; ce dispositif comprend en plus des moyens adaptés pour la diffusion des substances tels que par exemple une ou plusieurs têtes ou buses pour la vaporisation, nébulisation ou analogue de la substance par exemple sous forme de vapeur sèche évitant ainsi que des gouttes de produit ne se forment. Le dispositif selon l'invention comprend en outre un moteur qui actionne une pompe, celui-ci étant commandé par un variateur couplé à un microprocesseur ou analogue programmable afin de faire varier la vitesse de fonctionnement du moteur et donc de moduler l'intensité de diffusion des substances en fonction de l'horaire de fonctionnement.

Les moyens qui commandent le variateur et donc les caractéristiques de fonctionnement du moteur sont programmables afin de gérer l'intensité de diffusion au cours du fonctionnement. Ces moyens de commande du moteur peuvent en outre êtres couplés à des moyens qui permettent de capter des variations dans l'environnement par exemple une forte affluence ou des courants d'air, ceci afin d'adapter automatiquement la diffusion relativement à la variation de ces facteurs de l'environnement.

Dans un mode de réalisation préféré du dispositif selon l'invention, les moyens programmables qui commandent la gestion de l'intensité de diffusion peuvent être une puce ou analogue. Ces moyens programmables sont électroniques ou mécanique et peuvent être couplés à des moyens de commande manuels pour gérer l'intensité de diffusion au cours du temps. Ceci pour répondre à des variations des conditions de l'environnement inhabituelles et non prévues.

Le dispositif selon l'invention pour réaliser la fonction que l'on attend de lui est muni d'au moins un moyen par exemple du type vanateur qui commande la vitesse de fonctionnement du moteur et ou sa durée de fonctionnement et d'un microprocesseur ou analogue adapté pour être programmé et commander le variateur, ceci pour obtenir une intensité de diffusion désirée au moment souhaité.

Le dispositif possède également des moyens manuels autonomes pour agir sur le niveau de l'intensité de diffusion en réponse à des variations de l'environnement non prévues.

Le dispositif selon l'invention est adapté afin d'être alimenté en électricité de type secteur ou par des batteries.

Selon un autre mode de réalisation, le dispositif pourra être programmé à distance par tout moyen connu par exemple une télécommande. Dans une autre version du dispositif, celui-ci pourra être équipé de capteurs ou sondes pour la détection de variations dans les conditions de l'environnement notamment des mouvements, des variations de température, des concentrations de substances dans l'air, des variations d'hygrométrie, des flux d'air ou des variations d'intensité lumineuse.

Pour des raisons pratiques, le dispositif pourra se présenter sous la forme d'un appareil que l'on peut fixer sur un support de manière réversible de préférence ou être adapté pour être transporté.

Dans une variante de réalisation selon l'invention, le dispositif pourra également comporter un moyen de chauffage de la substance que l'on souhaite diffuser, ceci afin de faciliter la diffusion de la substance.

À la vue de la description qui précède, on comprend mieux que le dispositif selon l'invention remplit les objectifs que l'on attend de lui. L'homme du métier comprendra aisément que le dispositif pourra également être utilisé dans un ensemble plus complexe, sans sortir du périmètre de l'invention, où plusieurs dispositifs du même type pourront êtres utilisés et reliés entre eux afin de former un réseau pour une diffusion intelligente par exemple des odeurs dans des grands bâtiments, des réseaux de métro, des centres commerciaux etc.

## Revendications

1. Dispositif de diffusion de substances dans le milieu aérien ambiant comprenant au moins un moyen de stockage des ou de la substance à diffuser, des moyens adaptés pour la diffusion des substances, au moins un moteur pour le fonctionnement d'une pompe **caractérisé** en ce qu'il comprend des moyens de commande programmables et/ou non programmables pour gérer l'intensité de diffusion de substances au cours du fonctionnement.

2. Dispositif selon la revendication 1 caractérisé en ce qu'il comprend en outre des moyens manuels pour commander l'intensité de diffusion au cours du temps.

3. Dispositif selon l'une quelconque des revendications précédentes caractérisé en ce que les moyens de commande programmables de gestion de l'intensité de diffusion comprennent au moins un variateur et un microprocesseur adaptés pour la gestion de l'intensité de la diffusion.

4. Dispositif selon l'une quelconque des revendications précédentes caractérisé en ce que le microprocesseur commande le variateur.

5. Dispositif selon l'une quelconque des revendications précédentes caractérisé en ce que l'intensité de diffusion des substances pendant la plage de fonctionnement peut varier en fonction de l'horaire de fonctionnement.

6. Dispositif selon l'une quelconque des revendications précédentes caractérisé en ce que l'intensité de diffusion peut en outre être réglable manuellement.

7. Dispositif selon l'une quelconque des revendications précédentes caractérisé en ce que son fonctionnement peut être déclenché par des facteurs de l'environnement grâce à des moyens de type capteur ou sonde.

8. Dispositif selon la revendication 7 caractérisé en ce que les moyens de type capteur ou sonde sont sensibles notamment à des mouvements, des variations de température, à des concentrations de substances dans l'air, à des variations d'hygrométrie, des flux d'air ou des variations d'intensité lumineuse.

9. Dispositif selon la revendication 1 caractérisé en ce que la programmation peut être effectuée à distance.

10. Dispositif selon l'une quelconque des revendications précédentes caractérisé en ce que le dispositif peut être alimenté en électricité de type secteur ou par des batteries.

11. Dispositif selon la revendication 1 caractérisé en ce qu'il peut être fixé sur un support de manière réversible ou non ou en ce qu'il est transportable.

12. Dispositif selon la revendication 1 caractérisé en ce qu'il possède des moyens de chauffage des substances à diffuser pour faciliter la diffusion.
